# EUROPEAN PATENT APPLICATION

(11) **EP 4 768 502 A1**
(43) Date of publication of application: **01.07.2026**
(21) Application number: 24307326.9
(22) Date of filing: 27.12.2024
(51) Int. Cl.: C07K 16/10

(54) **ANTI-CORONAVIRUS ANTIBODY**

(71) Applicant: Ceva Santé Animale, 33500 Libourne (FR)
(72) Inventor: CHALLIER, Cécile, 33500 LIBOURNE (FR); VIGNE, Emmanuelle, 94240 L'HAY-LES-ROSES (FR)
(74) Representative: Vial, Lionel

(57) **Abstract**

The present invention relates to an anti-coronavirus antibody and its use thereof, particularly for the prevention or treatment of an infection caused by a coronavirus, especially by porcine epidemic diarrhea virus (PEDV). The anti-coronavirus antibody of the present invention comprises or consists of a first heavy chain variable domain comprising a CDR1 sequence comprising or consisting of FVAMA (SEQ ID NO: 1), a CDR2 sequence comprising or consisting of RISSTGTVTNYADSVKG (SEQ ID NO: 2) or a sequence having at least 90% identity with SEQ ID NO: 2, a CDR3 sequence comprising or consisting of VPGNLLP (SEQ ID NO: 3) or a sequence having at least 90% identity with SEQ ID NO: 3.

## Description

### Field of the invention

The present invention relates to an anti-coronavirus antibody and its use as a medicament, in particular for the prevention or treatment of an infection caused by a coronavirus, especially by porcine epidemic diarrhea virus (PEDV).

### Background

Porcine epidemic diarrhea (PED) is a diarrheal disease of swine caused by the porcine epidemic diarrhea virus (PEDV). It is characterized by acute watery diarrhea, dehydration and vomiting in swine of all ages and is especially fatal for neonatal and postweaning piglets. The disease was first described in Europe in the early seventies, and the PEDV virus was first isolated in Belgium and the United Kingdom in 1978. Since the nineties, several outbreaks have been reported in Asian countries, such as South Korea, China and Japan, and they have been characterized by relatively high mortality rates ranging from 50 to 95%, causing severe damage to the swine industry.

PEDV is a member of the Coronaviridae family and has a single-stranded, positive-sense RNA genome. PEDV mainly infects piglets and can infect several cell lines in vitro, such as Vero cells. The complete genome of PEDV comprises approximately 280,000 nucleotides with 5' and 3' ends containing untranslated regions (UTRs). The remaining genomic sequences contain seven open reading frames (ORFs) and encode four proteins: spike (S), envelope (E), membrane (M) and nucleoprotein (N). The spike protein of PEDV is a type one membrane glycoprotein consisting of the N terminal S1 and C terminal S2 subunits and plays an important role in mediating virus attachment and fusion to target cells. The spike protein contains a multidomain architecture and has been reported to bind to carbohydrate (sialic acid) and aminopeptidase N molecules in porcine cells.

Although there has been some success in developing a vaccine for PEDV, it is noteworthy that no effective vaccine is available in the market to protect newborn piglets. As such, an attenuated vaccine has been designed for use in sows to protect neonatal piglets in some Asian countries. However, the efficiency of the vaccine has not been assessed.

With the aim of providing effective antibodies for both diagnostic assays and treatment, Bao et al. (2019) AMB Expr 9:104 have reported expressing and purifying a PEDV-specific single-domain antibody that bound to the spike protein of PEDV in ELISA and stained the PEDV virus in Vero cells. However, it showed no neutralization activity on PEDV. Further, Qin et al. (2024) BMC Veterinary Research (2024) 20:336 disclosed a screening of specific nanobodies against the S1 protein from a phage display library obtained from immunized alpacas. High affinity nanobodies were selected and a multivalent tandem was constructed. However, there is no cross-reactivity over PEDV strains with the selected nanobodies.

Accordingly, there is still a need for alternative anti-PEDV antibodies, in particular with a neutralizing activity over the different types of PEDV strains.

### Summary of the invention

The present invention arises from the unexpected finding by the inventors of a single domain antibody capable of neutralizing various strains of PEDV, in particular both non-S-INDEL and S-INDEL PEDV strains. Advantageously, as demonstrated in example sections, the anti-coronavirus antibody has potent retention to PEDV strain even after enzymatic digestion, thus confirming the improved ability of the VHH according to the invention to be administered by oral route.

Accordingly, the present invention relates to an anti-coronavirus antibody comprising or consisting of a first heavy chain variable domain comprising:
- a CDR1 sequence comprising or consisting of FVAMA (SEQ ID NO: 1);
- a CDR2 sequence comprising or consisting of RISSTGTVTNYADSVKG (SEQ ID NO: 2) or a sequence having at least 90% identity with SEQ ID NO: 2, preferably having at least 95% identity with SEQ ID NO: 2, and more preferably having at least 98% identity with SEQ ID NO: 2.
- a CDR3 sequence comprising or consisting of VPGNLLP (SEQ ID NO: 3) or a sequence having at least 90% identity with SEQ ID NO: 3 preferably having at least 95% identity with SEQ ID NO: 3, and more preferably having at least 98% identity with SEQ ID NO: 3.

The present invention also relates to a nucleic acid encoding the anti-coronavirus antibody as defined above.

The present invention also relates to a vector comprising the nucleic acid as defined above.

The present invention also relates to the anti-coronavirus antibody as defined above, the nucleic acid as defined above, or the vector according as defined above, for use as a medicament in an individual.

The present invention also relates to the anti-coronavirus antibody as defined above, the nucleic acid as defined above, or the vector according as defined above, for use in the prevention or treatment of a coronavirus infection in an individual.

The present invention also relates to a method for preventing or treating a coronavirus infection in an individual, comprising administering to the individual a prophylactically or therapeutically effective amount of the anti-coronavirus antibody as defined above, the nucleic acid as defined above, or the vector according as defined above.

The present invention also relates to the anti-coronavirus antibody as defined above, the nucleic acid as defined above, or the vector according as defined above, for use in the prevention or treatment of a PEDV infection in an individual. Particularly, the PEDV infection is a S-indel or a non-S-indel PEDV infection.

The present invention also relates to a method for preventing or treating a PEDV infection, in particular a S-indel or non-S-indel PEDV infection, in an individual, comprising administering to the individual a prophylactically or therapeutically effective amount of the anti-coronavirus antibody as defined above, the nucleic acid as defined above, or the vector according as defined above.

The present invention also relates to a pharmaceutical composition comprising the anti-coronavirus antibody as defined above, the nucleic acid as defined above, or the vector according as defined above, as an active ingredient, optionally in association with at least one pharmaceutically acceptable carrier or excipient.

The present invention also relates to the use of the anti-coronavirus antibody as defined above, for the *in vitro* detection or quantification of a coronavirus, in particular a PEDV, in a biological sample.

The present invention also relates to a method, in particular an *in vitro* method, for diagnosing a coronavirus, in particular a PEDV, infection in an individual, comprising:
- contacting a biological sample taken from the individual with an anti-coronavirus antibody as defined above to detect or quantify a coronavirus, in particular a PEDV, in the biological sample;
- determining therefrom whether the individual is infected by a coronavirus, in particular a PEDV.

### Description of the invention

### Definitions

As a preliminary remark, it should be noted that the term "consisting of" means "constituted by", i.e. when an object "consists of" an element or several elements, the object cannot include other elements than those mentioned. In contrast, the term "comprising" means "including", "containing" or "encompassing", i.e. when an object "comprises" an element or elements, other elements than those mentioned can also be included in the object. In other words, when an object "comprises" an element or elements, it consists of the element(s) and possibly of other elements than these.

As understood here, the percentage of identity between two peptide sequences can be determined by performing an optimal alignment along the entire length of the sequences, determining the number of aligned positions for which the amino acids are identical in each sequence and dividing this number by the total number of amino acids in the longer of the two sequences. The optimal alignment is the one that gives the highest percentage of identity between the two sequences.

As intended herein, an *in vitro* method for diagnosing a disease is not a diagnostic method practiced on the human or animal body. It is carried out on biological samples that have been obtained from the human or animal body in a step that precedes, and that is not part of, the *in vitro* method as defined above.

As intended herein, a "biological sample" is a sample obtained from an individual that contains biological material, such as a bodily fluid.

Besides, as one of skilled in the art will well understand, the expressions "substance or composition for use in the prevention or treatment of a disease" is equivalent to the expression "substance or composition for use in a method for the prevention or treatment of a disease".

As intended herein, an antibody, commonly referred to as an immunoglobulin, is a protein produced predominantly by plasma cells that is used by the immune system to identify and bind to non-self antigens such as bacteria and viruses. An antibody generally comprises heavy and light chains, each consisting of constant and variable regions. The constant region determines the class or isotype of the antibody and mediates interaction with effector cells and complement proteins, while the variable region is involved in antigen binding. The variable regions of the heavy and light chains come together to form the antigen-binding site, characterized by three hypervariable loops known as complementarity-determining regions (CDRs). These regions, CDR1, CDR2, and CDR3, exhibit the most variability and are crucial for the antibody's antigen specificity and affinity. HCDR1, HCDR2 and HCDR3 refer to the CDRs of the heavy chain while LCDR1, LCDR2 and LCDR3 refer to the light chain CDRs. The remaining parts of the variable regions are more conserved and form the so-called framework regions, which provide structural support for the CDRs.

Furthermore, antibodies as intended herein include variations such as:
- Single Domain Antibodies (sdAb), also known as VHHs or nanobodies when derived from camelids, these consist of a single monomeric variable domain, comparable to VH, with a distinctive structure that enables stability and functionality in various conditions. Their small size facilitates tissue penetration and the potential for modular fusion to other molecular entities.
- Single-chain Variable Fragment (scFv), which comprise the variable regions of both the heavy (VH) and light (VL) chains connected via a flexible peptide linker, scFvs maintain binding specificity and offer the advantage of reduced molecular size. This configuration enables versatile applications, including the generation of bispecific constructs and ease of production in microbial systems.
- Diabodies, which are constructed from scFv domains connected without a linker sufficiently long to prevent intrachain pairing, forcing interchain dimerization to create bivalent binding sites. They offer enhanced avidity and selectivity, desirable in applications requiring crosslinking or bispecific targeting.
- F(ab')2 and Fab Fragments. The F(ab')2 fragment consists of two antigen-binding Fab portions linked by disulfide bonds, resulting from the enzymatic cleavage of an intact IgG molecule. Each Fab region contains one VH and one VL domain linked to constant regions (CH1 and CL), retaining the antigen-binding functionality akin to a full antibody but without the Fc region. A Fab fragment is generated by papain cleavage, yielding two identical antigen-binding regions without the need for an Fc-mediated effector function, which is suited for therapeutic applications where only blocking activity is desired.
- Fab' Fragments. A Fab' fragment is similar to a Fab but features additional amino acids that facilitate linkage to other molecules or surfaces and potentially create a F(ab')2 upon forming disulfide bonds. It provides an interaction site for additional moieties or stable immobilization.

These variations provide several advantages, including improved pharmacokinetics, reduced immunogenicity, ease of recombinant production, and enablement of diverse therapeutic and diagnostic applications.

As intended herein an epitope is a specific region or surface of an antigen molecule to which an antibody binds through its paratope, which is formed by its CDRs.

As intended herein a neutralizing antibody is an immunoglobulin capable of binding to a specific antigen of a pathogen, such as a viral protein, with sufficient affinity to interfere with and prevent the biological activity or infectivity of the pathogen. By attaching to a virus, the neutralizing antibody notably impedes or neutralizes its ability to infect host cells, thereby inhibiting its replication and providing a protective immune response. These antibodies are often characterized by their ability to block virus entry into cells or disrupt the function of viral proteins essential for replication or pathogenesis.

As intended herein coronaviruses are a group of positive-sense single-stranded RNA viruses belonging to the family Coronaviridae and the order *Nidovirales*. These viruses primarily affect mammals and birds and are characterized by their ability to cause illnesses ranging from mild respiratory infections to severe acute respiratory syndromes. Coronaviruses are spherical, enveloped viruses featuring prominent spike (S) glycoproteins that protrude from their surface, providing a crown-like appearance under electron microscopy, from which their name is derived.

Coronaviruses that predominantly target non-human animals include:
- Porcine Epidemic Diarrhea Virus (PEDV): Affects pigs, characterized by severe diarrhea and dehydration, especially in piglets.
- Transmissible Gastroenteritis Virus (TGEV): Also targets pigs, causing gastroenteritis with a high mortality rate in young piglets.
- Porcine Respiratory Coronavirus (PRCV): Affects pigs and is associated with mild respiratory symptoms.
- Bovine Coronavirus (BCoV): Impacts cattle, leading to respiratory infections and diarrhea, particularly in calves.
- Avian Infectious Bronchitis Virus (IBV): Predominantly infects poultry, causing respiratory illnesses, decreased egg production, and poor quality of eggs.
- Canine Coronavirus (CCoV): Infects dogs, resulting in mild gastroenteritis, especially in young puppies.
- Feline Infectious Peritonitis Virus (FIPV): Affects cats, often resulting in fatal systemic disease.
- Murine Hepatitis Virus (MHV): Impacts mice, leading to various disease manifestations depending on the virus strain and host genetic factors.

Porcine epidemic diarrhea virus (PEDV) induces porcine epidemic diarrhea (PED) which manifests itself primarily in swine and exhibits various clinical symptoms. Affected pigs experience acute watery diarrhea, which is profuse and may lead to significant dehydration and electrolyte imbalance. Swine across all ages can contract the disease, but it is especially lethal in neonatal and postweaning piglets due to their increased susceptibility to dehydration. Alongside diarrhea, vomiting is frequently observed, further exacerbating fluid loss and leading to rapid onset of clinical distress. The disease can result in severe cases of weakness and, ultimately, can be fatal if not adequately managed, particularly in younger piglets.

Two different genogroups of PEDV, S INDEL [PEDV variant containing multiple deletions and insertions in the S1 subunit of the spike (S) protein, G1b] and non-S INDEL (G2b) strains were detected during the diarrheal disease outbreak in US swine in 2013-2014. Although the non-S INDEL PEDV was highly virulent and the S INDEL PEDV caused milder disease, the latter has the capacity to cause illness in a high number of piglets on farms with low biosecurity and herd immunity (Jung et al. 2020, Virus Res. Sep:286:198045).

As intended herein non-S-indel strains, relates to coronavirus strains which lack specific insertions/deletions in the spike protein gene and are implicated in recent outbreaks with distinct virulence profiles. For a comprehensive review on non-S-indel strains of Porcine Epidemic Diarrhea Virus (PEDV), one may refer to the article by Chen et al. (2017) Journal of Clinical Microbiology 55:2331-2341. This article provides a detailed examination of various PEDV strains, including the non-S-indel variants, and discusses their genetic characteristics, pathological impacts, and geographical distribution, as observed during major outbreak events.

As intended herein, a pharmaceutical composition or a medicament is a formulation comprising an active ingredient, such as an antibody, nucleic acid, or vector, optionally in combination with one or more pharmaceutically acceptable carriers, excipients, or diluents. These additional components serve to ensure the stability, bioavailability, and delivery of the active ingredient when administered to an individual. The carriers and excipients are selected based on their compatibility with the active ingredient and the intended mode of administration, which may include but is not limited to, oral, intravenous, intramuscular, or subcutaneous routes.

As intended herein passive immunotherapy refers to a treatment modality that involves the administration of pre-formed antibodies to an individual. These antibodies, which can be derived from human or animal sources, or synthetically produced in laboratories, confer immediate protection against a specific antigen, pathogen, or toxin without the host having to mount their own immune response. Passive immunotherapy can be particularly useful in providing short-term protection or in managing acute infections or conditions where the body's immune system is either compromised or needs to respond rapidly to prevent progression of disease. The antibodies utilized in passive immunotherapy perform their function by directly neutralizing pathogens, targeting them for destruction by immune cells, or blocking their ability to enter host cells. This approach is distinct from active immunization strategies, such as vaccines, which stimulate the host's immune system to generate a long-lasting immune response. Passive immunotherapy can be employed for either prophylactic or therapeutic purposes. For prevention, passive immunotherapy can be administered prior to, or immediately after exposure to a pathogen, thereby providing immediate immunity or reducing the likelihood of infection. For treatment, passive immunotherapy is used during the course of an infection to mitigate its severity, support the immune system's efforts to clear the pathogen, and hasten recovery.

### Antibody

Preferably, the first heavy chain variable domain as defined above comprises or consists of 
EVQLVESGGGLVQAGGSLRLSCAASGSLLSFVAMAWYRQVPGKEREVVARISSTGTVTNYADS
VKGRFTISRDNAKKTLDLQMNSLKPEDTAVYYCNRVPGNLLPWGQGTQVTVSS (SEQ ID NO: 4)
 or of a sequence having at least 90% identity with SEQ ID NO: 4, preferably having at least 95% identity with SEQ ID NO: 4, and more preferably having at least 98% identity with SEQ ID NO: 4.

Preferably, the first heavy chain variable domain as defined above is a VHH or a nanobody.

Preferably, the anti-coronavirus antibody as defined above comprises two or more first heavy chain variable domains.

Preferably, the anti-coronavirus antibody as defined above further comprises at least one second heavy chain variable domain.

Preferably, the second heavy chain variable domain as defined above is a VHH or a nanobody.

Preferably, the second heavy chain variable domain is directed against an epitope of the coronavirus spike protein which is different from the epitope of the first heavy chain variable domain.

Preferably, the anti-coronavirus antibody comprises or consists of 
EVQLVESGGGLVQAGGSLRLSCAASGSLLSFVAMAWYRQVPGKEREVVARISSTGTVTNYADS
VKGRFTISRDNAKKTLDLQMNSLKPEDTAVYYCNRVPGNLLPWGQGTQVTVSSGSGSGSGSGSE
VQLVESGGGLVQAGGSLRLSCAASGSLLSFVAMAWYRQVPGKEREVVARISSTGTVTNYADSV
KGRFTISRDNAKKTLDLQMNSLKPEDTAVYYCNRVPGNLLPWGQGTQVTVSS (SEQ ID NO: 5) or
 of a sequence having at least 90% identity with SEQ ID NO: 5, preferably having at least 95% identity with SEQ ID NO: 5, and more preferably having at least 98% identity with SEQ ID NO: 5.

Preferably, the anti-coronavirus antibody as defined above is a neutralizing antibody.

Preferably, the anti-coronavirus antibody as defined above is an anti-PEDV antibody, in particular an anti-non-S-indel PEDV antibody.

Preferably, the anti-coronavirus antibody as defined above is capable of binding, in particular of neutralizing, several, more preferably essentially all, PEDV strains. More preferably, the anti-coronavirus antibody is capable of binding, in particular of neutralizing, S-indel and non-S-indel strains of PEDV.

Some of the notable PEDV strains that have been identified and studied include:
- Classical strains such as CV777, known for their historical significance in early outbreaks;
- GDU, a strain identified for specific research in antibody neutralization;
- DR13, often used as a reference strain in experimental infections.

### Nucleic acid

The nucleic acid as defined above may be deoxyribonucleic acid (DNA) or ribonucleic acid (RNA). This nucleic acid may be double-stranded or single-stranded and is capable of undergoing transcription and/or translation processes within a host cell to produce the anti-coronavirus antibody, thereby facilitating its expression and resulting biological activity.

The nucleic acid may comprise atypical nucleotides, such as pseudouridine, which can be incorporated into the RNA. Pseudouridine is a modified nucleotide characterized by the isomerization of uridine, enhancing the stability and translation efficiency of the RNA. This modification is particularly advantageous in contexts where enhanced functionality or stability of the expressed antibody is desired.

The nucleic acid may comprise modifications that enhance its stability or expression. Such modifications can include the addition of a poly(A) tail, which serves to increase mRNA stability and translation efficiency, and a 5' 7-methylguanylate cap (5'mG), which enhances mRNA stability, aids in ribosomal recognition, and protects the RNA from degradation. These modifications collectively contribute to the improved functionality and persistence of the mRNA within a cellular environment, thereby amplifying the synthesis of the anti-coronavirus antibody.

The nucleic acid as defined above may be comprised in a vector. Use of vectors ensures that the nucleic acid is adequately protected, delivered to the appropriate cell type, and expressed efficiently to yield the desired therapeutic effect. Exemplary vectors include viral vectors such as adenovirus or lentivirus, pseudoviruses designed for enhanced transduction efficiency while lacking pathogenicity, and lipid nanoparticles (LNP), which offer a non-viral means of delivery. These vectors are selected based on their compatibility with the intended therapeutic application and the target host organism.

### Individual

The individual as defined above is a human or a non-human animal.

Preferably, the individual is a non-human animal. It can be a mammal, a marsupial or a bird.

Preferably, the individual as defined above is a livestock or a pet.

Examples of livestock according to the invention include porcine (pigs), bovine (cattle), ovine (sheep), caprine (goats), equine (horses), galline (chickens), meleagrine (turkeys), anserine (geese), anatine (ducks), bubaline (buffalo).

Examples of pets according to the invention include dogs, cats, rabbits or ferrets.

More preferably, the individual as defined above is a porcine. Most preferably, the individual is a young porcine, especially a piglet, in particular aged less than 1 year, 6 months, 3 months, 2 months, 1 month, 3 weeks, 2 weeks, 1 week, 6 days, 5 days, 4 days, 3 days or 2 days.

### Treatment

The anti-coronavirus antibody, the nucleic acid, the vector, the medicament and the pharmaceutical composition as defined above are useful for passive immunotherapy.

As such the anti-coronavirus antibody, the nucleic acid, the vector, the medicament and the pharmaceutical composition as defined above can be used as a vaccine or a serum.

The anti-coronavirus antibody, the nucleic acid, the vector, the medicament and the pharmaceutical composition as defined above may be formulated as a solution for injection, comprising the antibody in a pharmaceutically acceptable buffer, such as phosphate-buffered saline (PBS), with or without preservatives such as benzyl alcohol. The solution can be further supplemented with stabilizers like arginine or trehalose to maintain antibody activity. The anti-coronavirus antibody, the nucleic acid, the vector, the medicament and the pharmaceutical composition as defined above can also be processed into a lyophilized powder, facilitating long-term storage stability. This form may include cryoprotectants or lyoprotectants, such as sucrose or mannitol, and can be reconstituted in a suitable diluent, such as sterile water for injection or a buffered saline solution, prior to administration. The anti-coronavirus antibody, the nucleic acid, the vector, the medicament and the pharmaceutical composition as defined above can also be formulated as an oral formulation. In that when the stability of antibodies, nucleic acids or vectors in the gastrointestinal tract is a consideration, encapsulation techniques, such as enteric-coated capsules or microencapsulation, may be employed to protect them. This formulation would be designed to release the antibody in the intestine, potentially in combination with absorption-enhancing agents. The anti-coronavirus antibody, the nucleic acid, the vector, the medicament and the pharmaceutical composition as defined above antibody may be formulated as an aerosol for inhalation, utilizing aerosolized delivery systems. The antibody is typically solubilized in an aqueous carrier suitable for nebulization, potentially with the inclusion of agents to improve dispersion or particle size, such as surfactants. The anti-coronavirus antibody, the nucleic acid, the vector, the medicament and the pharmaceutical composition as defined above may be incorporated into an intranasal spray formulation. This form may comprise the antibody, the nucleic acid or the vector in a buffered solution with osmolarity-adjusting agents and mucoadhesive polymers designed to enhance retention and absorption through the nasal mucosa.

The anti-coronavirus antibody, the nucleic acid, the vector, the medicament and the pharmaceutical composition as defined above can be administered through several routes of administration. As such, the anti-coronavirus antibody, the nucleic acid, the vector, the medicament and the pharmaceutical composition as defined above may be administered via intravenous injection (IV route), allowing the antibody to enter directly into the bloodstream for systemic circulation. This route is suitable for achieving rapid therapeutic levels in plasma. The anti-coronavirus antibody, the nucleic acid, the vector, the medicament and the pharmaceutical composition as defined above can also be injected into muscle tissue (muscular route), using a formulation that permits slow absorption into systemic circulation. This method usually provides a sustained release compared to intravenous administration. The anti-coronavirus antibody, the nucleic acid, the vector, the medicament and the pharmaceutical composition as defined above can also be administered by the subcutaneous (SC) route. Subcutaneous administration involves injecting the antibody under the skin, allowing for gradual absorption into the bloodstream. This route is advantageous for periodic dosing and patient self-administration. The anti-coronavirus antibody, the nucleic acid, the vector, the medicament and the pharmaceutical composition as defined above can also be administered by the inhalation route. The antibody formulation may be delivered through inhalation, using nebulizers or inhalers, providing direct delivery to the respiratory tract. This route can be efficient for treating or preventing respiratory infections caused by coronaviruses. The anti-coronavirus antibody, the nucleic acid, the vector, the medicament and the pharmaceutical composition as defined above can also be administered by the oral route. The anti-coronavirus antibody, the nucleic acid, the vector, the medicament and the pharmaceutical composition as defined above can also be administered by the intranasal route, for instance using sprays or drops, to target mucosal surfaces in the nasal passages for enhanced retention and absorption.

Preferably, the anti-coronavirus antibody, the nucleic acid, the vector, the medicament and the pharmaceutical composition as defined above is administered by the oral route.

### Doses

The doses can be established by routine experimentation and depends on the neutralizing properties of the anti-coronavirus antibody chosen but also on the required level of protection.

The anti-coronavirus antibody, the nucleic acid, the vector, the medicament and the pharmaceutical composition as defined above can be used at a dose comprised between 0.2 mg/kg and 5 mg/kg, preferably at a dose comprised between 0.3 mg/kg and 4 mg/kg, and more preferably at a dose comprised between 0.5 mg/kg and 3 mg/kg.

### Detection

Biological samples as defined above may include, but are not limited to, blood, serum, plasma, saliva, nasal swabs, throat swabs, urine, feces, cerebrospinal fluid, tissue biopsies, and bronchoalveolar lavage fluid.

The anti-coronavirus antibody as defined above can be used to detect or quantify a coronavirus, in particular a PEDV, in the biological sample according to several methods well know to a person of skill in the art.

By way of example, the anti-coronavirus antibody as defined above may be utilized in an ELISA. In this method, the sample is added to a well coated with the anti-coronavirus antibody. If a coronavirus, in particular a PEDV, or a part thereof, is present, it will bind to the antibody. A secondary antibody conjugated to an enzyme, specific to the coronavirus, in particular the PEDV, is then added and binds to any captured virus. A substrate is introduced, and the presence of enzyme activity can be measured by a colorimetric change, indicating the presence of a coronavirus, in particular a PEDV, or a part thereof.

The disclosure is in the hereinafter provided paragraphs described by way of example, in relation to the figures. The figures are provided for a better understanding of the example embodiments and are not intended to limit the present disclosure.

### Brief description of the figures

Figure 1 depicts multiple curves corresponding to concentration-dependent cross reactivity measured at OD 450nm with non-S-INDEL and S-INDEL strains of PEDV for VHH candidate 5.
Figure 2 depicts multiple curves corresponding to concentration-dependent cross reactivity measured at OD 450nm with non-S-INDEL and S-INDEL strains of PEDV for VHH candidate 7.
Figures 3 represents ability of the VHH candidate 7 (A) and VHH7-5GS-VHH7 (B) to bind GDU-S pike protein, via ELISA, after being submitted to pancreatin digestion.

### EXAMPLES

### Example 1: Production and selection of VHH candidates

The spike glycoprotein (S) of PEDV on the surface of the was selected as the main target of neutralizing antibodies.

### 1. Antigen production and quality

### • Production

The full-length spike (S) protein of the GDU strain of porcine epidemic diarrhea virus (GDU-PEDV) has been produced in HEK cells to ensure a correct trimer conformation and glycosylation of the protein. Overall, 10 mg of protein were obtained.

### • Quality control

### SDS-Page QC of control mAb

The purity and integrity of a control anti-spike mouse mAb has been analysed for the experiments.

Briefly, 1 µg of antibody were analyzed by SDG-PAGE under reducing and non-reducing conditions. The results show that the control mAb runs at the expected molecular weight (approximately 150kDa) under non-reducing conditions and at 50kDa (heavy chain) and 25kDa (light chain) under reducing conditions. It was therefore used as control.

### Biotinylation of GDU-S protein

The GDU-PEDV spike (GDU-S) protein to be used in phase display selections and screening activities was randomly biotinylated.

Briefly, 150 µg of GDU-S protein was conjugated using 10-fold molar excess of biotin using EZ-Link^{®} KHS-Biotin (cat. Nr. 21336 Thermo Scientific). Then, free biotin was removed, and buffer exchanged to 1xPBS buffer with Amicon Ultra-0.5 3K desalting column.

The biotinylated protein information and quantification are summarized below:
- Lysine (K) count: 41
- Length (aa): 1385
- A260/280: 0.645
- A280nm: 0.548
- Concentration (mg/ml): 0.548
- E extinction coefficient 0.1% (1g/L at 280nm) : 0.9
- Final concentration adjusted (mg/ml): 0.609
- Final volume (µL): 130
- Total amount recovered (µg): 79.17
- Yield (%): 53

### SDS-Page QC of GDU-S spike protein

The purity and integrity of biotinylated and non-biotinylated GDU-S protein was compared via SDS-PAGE using 1µg of protein under reducing and non-reducing conditions.

The GDU-S protein presented the expected profile under reducing and non-reducing conditions (151kDa). The biotinylated protein had a similar SDS-PAGE profile than the non-biotinylated protein, confirming that biotin conjugation did not affect protein integrity.

### Western Blot QC of bio-GDU-S protein

The presence of biotin was verified on biotinylated GDU-S protein via Western-Blot.

Briefly, 200 ng of the protein were separated via SDS-PAGE, transferred to a cellulose membrane and analyzed by Westen-Blot using ExtrAvidin-HRP as a detection reagent at 1 :2500 dilution. Biotin was successfully detected by the ExtrAvidin-HRP reagent under both reducing and non-reducing conditions.

### ELISA QC of GDU-S protein

Quality control of the biotinylated and non-biotinylated GDU-S protein was verified via titration binding ELISA, using the control anti-spike mouse mAb antibody and ExtrAvidin-HRP reagent for detection.

A full titration curve was obtained for the biotinylated GDU-S protein captured by neutravidin when detected with the control mAb, indicating that biotin was successfully conjugated. A full titration curve was also obtained for directly coated GDU-S protein, before and after biotinylation, when detected with the control mAb Control. Both proteins exhibited a similar profile indicating that biotinylation did not affect the availability of epitopes of the GDU-S protein.

Further, no binding was detected for the blank control (no antigen), nor for the secondary antibody controls.

The foregoing results showed that the quality and the purity of the GDU-S protein produced in HEK cells was satisfactory and that it could be used for llama immunization.

### 2. Llama immunization

Two llamas were immunized with the GDU-S protein obtained as described above. The llamas received subcutaneous (SC) injections of GDU-S protein every 7 days for 6 weeks according to the immunization scheme presented in Table 1:

**Table 1**

| | **Llama A** | **Llama B** | **Administration route** | **Adjuvant (adjuvant: GDU-S)** | **Blood collection** |
|---|---|---|---|---|---|
| **Immunization day** | **Dose of GDU-S** | | | | |
| 0 | 100 µg | 100 µg | SC | IFA (1:1) | 20 mL (pre-immune) |
| 7 | 100 µg | 100 µg | SC | IFA (1:1) | N/A |
| 14 | 50 µg | 50 µg | SC | IFA (1:1) | N/A |
| 21 | 50 µg | 50 µg | SC | IFA (1:1) | N/A |
| 28 | 50 µg | 50 µg | SC | IFA (1:1) | N/A |
| 35 | 50 µg | 50 µg | SC | IFA (1:1) | N/A |
| 39 | N/A | N/A | N/A | N/A | 400 mL (post-immune) |

| | | | | | |
|---|---|---|---|---|---|
| N/A - not applicable / IFA - incomplete Freund's Adjuvant / SC - Subcutaneous injection / 1:1 - v/v | | | | | |

The two llamas' immune responses were evaluated by ELISA by titrating the pre-immune (background) and post-immune serum samples on GDU-S protein (the optical density (O.D.) values at 450 nm were measured). Both llamas showed clear and similar immune responses against the GDU-S protein.

### 3. VHH libraries generation

Two VHH phase display libraries were generated from peripheral blood lymphocytes (PBLs) collected from each llama according to standard procedures.

### • RNA extraction and quality control

Total RNA was extracted from ethanol precipitates of PBL samples.

A high integrity was observed for total RNA purified from PBLs of the two llamas as shown by the 18S and 28S RNA bands observed in the bioanalyser QC. Further, RNA integrity number (RIN) was above 9 for both immunized llamas indicating high integrity of the RNA. Finally, high ratios Abs (260/230) and Abs (260/280) above 1.7 were observed for all llamas also indicating good purity.

### • cDNA synthesis

cDNA was generated from total RNA using the SuperScript^{®} III first-Strand cDNA kit (Invitrogen, Cat. Nr. 1808-051). A volume of 160 µl of cDNA was obtained per llamas and was used for amplifying VHH coding sequences.

### • PCR amplification

The VHH coding sequences were amplified by polymerase chain reaction (PCR) using untagged primers for constructing single domain antibody libraries.

To do so, a first step of PCR amplification performed using cDNA, non-tagged forward and reverse primers annealing at VH leader and CH2 regions, respectively, and Expand High Fidelity PCR system (Roche, Cat. Nr. 4738276001). The 700 bp band was extracted and purified using the NucleoSpin^{®} Gel and PCR Clean-up (Macherey Nagel) following manufacturer's instructions. A second amplification introducing restriction enzyme sites was performed using the purified primary PCR product, tagged forward and reverse primers and Expand High Fidelity PCR system. Finally, 5µl of each nested PCR amplification reaction from each llama, was run in a 1% agarose ge!/1xTAE and Gene Ruler DNA ladder was used as reference.

### • VHH genes cloning

The VHH coding sequences from the two single domain antibodies libraries were cloned in a phagemid vector.

Briefly, the nested PCR VHH products were digested with two different restriction endonuclease enzymes and the digested product was purified with NucleoSpin^{®} Gel PCR Clean-up Kit. The phagemid vector was digested by the same restriction endonuclease enzymes and ligated with the digested VHH PCR products, and a ligation reaction was incubated O/N at 16°C.

### • Final transformation

Briefly, per library, each purified ligation was used to transform ECC TG1 cells (Lucigen, Cat. Nr. 60502-2). The library ligation and TG1 cell mix were aliquoted into pre-chilled 0.1cm cuvettes (Bio-Rad, Cat. Nr. 1652089) and electroporated using program EC1 (Bio-Rad Pulser). Time constant varied between 5-5.4 msec.

The library size and VHH insert percentage determination from VHH final transformation are presented in Table 2 below:

**Table 2**

| **Llama ID** | **Library name** | **Library size (CFU)** | **VHH insert** (**%**) |
|---|---|---|---|
| Llama A | Library A | 1.14E+08 | 95 |
| Llama B | Library B | A.20E+08 | 100 |

Two llama VHH libraries with a size above 1.1E+08 CFU and a VHH insert percentage above 95% were generated. For further processing, the purified phage libraries that have been obtained are stored at -80°C until next step.

### 4. Phage display in-solution selections

A phage display selection has been performed following standard procedures known in the art with the two llama VHH libraries produced as detailed in previous sections. To select anti-GDU-S VHHs a first round (R1) of in-solution selections using biotinylated PEDV-GDU-S protein pre-capture on streptavidin magnetic coated beads was performed in a KingFisherTM Flew System. An enrichment of 3 and 98-fold compared to background (0 nM of protein) were observed for outputs selected with the libraries A and B respectively.

A second round (R2) of in-solutions selections following the same procedure as the first round. An enrichment up to 2740-fold and 60-fold compared to background (0 nM of protein) were observed for the outputs selected with R1 phage from the libraries A and B respectively.

Finally, a third round (R3) of in-solutions selections was done and enrichments up to 350-fold and 416-fold compared to background (0 nM of protein) were observed for the outputs selected with R2 phage from libraries A and B respectively.

### 5. Primary screening

Master plates were generated using enriched outputs from both libraries.

Binding of monoclonal VHH periplasmic extracts (P.E.) from the master plates was assessed by ELISA, using biotinylated GDU-S Spike protein captured on neutravidin. Due to the low hit-rates observed, possibly related a certain instability of the biotinylated protein, the screening was completed on directly coated GDU-S protein. A total of 288 positive clones were identified.

### 6. Sequencing and sequence analysis

All positive clones were sequenced to determine sequence diversity.

The sequence analysis showed a high diversity with a total of 140 VHH unique sequences and 58 HCDR3 sequences identified for both libraries.

The clones were screened as monoclonal VHH periplasmic extracts (P.E.) by binding ELISA on directly coated GDU-S protein at pH 4.5 and 7.4.

From the 140 clones screened, 121 and 58 clones showed positive binding to GDU-S spike protein at pH 7.4 and 4.5, respectively, with OD values above 0.1 when blank was subtracted.

The same 140 unique VHH clones were also screened for off-rate determination using the Biacore T200. Briefly, the GDU-S spike protein was immobilized on the surface of a CM5 chip and the VHHs allowed to bind in solution. A total of 102 VHH clones showed specific association to GDU-S protein (RU max > 10), with kd values between 3.98E-02 and 7.13E-05 kd(s-1). The remaining 38 VHH clones showed RU values below 10 RUs on GDU-S protein and therefore the off-rate was not determined.

Based on these results, the inventors selected the 10 most promising VHH candidates for further analysis and characterization.

### Example 2: Characterization of selected VHH candidates

### 1. Production and purification of the VHH candidates

The His-tagged candidates have been produced and affinity purified. The production yield and the purity of the VHH candidates are presented in Table 3 below:

**Table 3**

| **VHH candidate** | **MW (kDA)** | **Concentration (mg/ml)** | **Protein quantity (mg)** | **Purity (%)** | **LPS (EU/mg)** |
|---|---|---|---|---|---|
| 1 | 14.13 | 0.83 | 5.32 | 92 | 0.09 |
| 2 | 13.91 | 0.54 | 2.36 | 88 | 0.70 |
| 3 | 15.50 | 1.00 | 2.41 | 95 | 0.36 |
| 4 | 14.11 | 0.84 | 5.42 | 94 | 0.09 |
| 5 | 14.56 | 0.55 | 1.97 | 96 | 0.46 |
| 6 | 13.87 | 0.84 | 2.52 | 99 | <0.05 |
| 7 | 13.44 | 0.48 | 1.50 | 94 | 0.31 |
| 8 | 14.22 | 0.68 | 4.50 | 98 | 0.58 |
| 9 | 14.62 | 0.60 | 3.86 | 94 | 0.63 |
| 10 | 13.46 | 1.26 | 7.50 | 98 | 0.07 |
| Control | 13.92 | 0.62 | 3.62 | 97 | 0.83 |

The produced panel had variable yields, ranging from 60 to 300 mg/L and with a purity above 92 % as determined by SEC-HPLC, except for VHH candidate 2. An irrelevant control VHH obtained from VHH candidates 2, 3 and 5 was also produced and purified with a yield of 145 mg/L, to be used as control in the characterization assays. Briefly, the control VHH was obtained with the same protocol as those described in Example 1, except that the llamas were immunized with a GDU-E protein instead of GDU-S protein.

### 2. Affinity determination

The specificity of the VHH candidates to the GDU-S protein was confirmed by SPR and binding ELISA to determine their affinity and to identify different epitopes.

### • Affinity determination by SPR

The affinity of the VHH candidates to GDU-S spike protein was determined by SPR using Biacore T200 in single cycle kinetics (SCK) at pH 7.4 with the following specifications:
- Antigen: GDU-S spike protein was immobilized at 1156 RUs on Sensor Chip CM5 (Cytivia, ref. 29149603).
- Sample: Two-fold sample dilutions of the clones starting at 200 to 12.5 nM or 20 to 1.3 nMin 1xHBS-EP+pH 7.4, at 30µl/min for2 minutes followed by off-rate wash for 1 min. Off-rate wash extended to 5 min after the final VHH injection in each cycle.
- Control: the control VHH.
- Regeneration: one injection of 1 M NaCl, 1 mM Glycine pH 1.5 followed by one injection of 1xHBS-EP+ pH 7.4
- Biacore buffer: 1xHBS-EP+ buffer pH 7.4 (GE healthcare, Cat. Nr. BR-1006-69)
- Analysis: Affinity and RU max calculated using the Biacore evolution software.

A 60-70 RU response was observed when the control VHH was used at 200 nM at the beginning and at the end of the experiment, therefore validating the chip integrity throughout the assay. Further, regeneration of the chip surface was complete for all cycles and no binding of the control VHH was observed.

The kinetics parameters determined using the 1:1 binding fitting applied using the Kinetic/affinity of VHH binding to GDU-S spike protein are presented in Table 4 below:

**Table 4**

| VHH Candidate | Concentration range | Kinetics Chi² (RU²) | ka (1 /Ms) | Kd (1/s) | Rmax (RU) | Kd (nM) |
|---|---|---|---|---|---|---|
| 1 | 200 - 12.5 | 0.33 | 9.37E+04 | 4.94E-04 | 36.19 | 5.27 |
| 2 | 200 - 12.5 | 0.72 | 1.21E+05 | 4.32E-03 | 34.27 | 35.59 |
| 3 | 200 - 12.5 | N.D. | N.D. | N.D. | N.D. | N.D. |
| 4 | 20 - 1.25 | 0.03 | 3.63E+06 | 7.74E-04 | 30.31 | 0.21 |
| 5 | 20 - 1.25 | 0.11 | 2.25E+06 | 3.43E-03 | 33.11 | 1.53 |
| 6 | 20 - 1.25 | 0.09 | 1.07E+06 | 5.98E-04 | 24.84 | 0.56 |
| 7 | 20 - 1.25 | 0.12 | 2.90E+06 | 3.68E-03 | 25.75 | 1.27 |
| 8 | 200 - 12.5 | 0.33 | 1.02E+05 | 7.74E-04 | 34.43 | 7.58 |
| 9 | 20 - 1.25 | 0.09 | 2.43E+06 | 3.17E-03 | 32.89 | 1.31 |
| 10 | 200 - 12.5 | 0.09 | 1.08E+05 | 5.75E-04 | 23.32 | 5.32 |

As can be seen, 9 VHHs showed an association and dissociation to GDU-S protein with Kd values ranging from 0.21-35.6 nM. Unfortunately, VHH candidate 3 did not show specific binding to the GDU-S protein.

### • EC50 by ELISA at pH 7.4 and pH 4.4

The EC50 of the VHH candidates was determined by binding ELISA on GDU-S protein at pH 4.4 and 7.4, using the following specifications:
- Coating: GDU-S protein at 30µl/ml in 1x PBS buffer
- Blocking: 4% skimmed milk in 1x PBS
- Sample: three-fold serial dilutions of VHH candiates at 300 nM down to 0.0002 nM in 1% skimmed milk in 1x PBS pH 7.4 pr 1x CPA pH 4.4.
- Detection: anti-histidine-HRP (Miltenyi Biotec, Cat. Nr. 130-092-783) at 1:500 dilution in 1% skimmed milk in 1x PBS pH 7.4 pr 1x CPA pH 4.4.
   ∘ Coating control: control VHH at 5 µg/ml in 1% skimmed milk in 1x PBS pH 7.4 pr 1x CPA pH 4.4 followed by anti-mouse IgG-HRP (JIR, Cat. Nr. 715-035-150) at 0.16 pg/ml in 1x PBS pH 7.4 pr 1x CPA pH 4.4.
- Development: TMB (eBioscience, Cat. 00-4201-56)
- Stop reaction: H2SO4 (Fisher Chemical, Cat. J8430/15)

The results are presented in Table 5 below:

**Table 5**

| **VHH Candidate** | **EC50 (nM)** | |
|---|---|---|
| | **pH 7.4** | **pH 4.4** |
| 1 | 3.38 | 20.43 |
| 2 | 16.39 | N.D. |
| 3 | N.D. | N.D |
| 4 | 0.68 | N.D. |
| 5 | 1.93 | N.D. |
| 6 | 0.76 | N.D. |
| 7 | 1.94 | N.D. |
| 8 | 3.13 | 22.13 |
| 9 | 1.23 | N.D. |
| 10 | 4.44 | N.D. |

Specific binding was observed for all clones tested at pH 7.4 except forVHH candidate 3. VHH candidates 1 and 8 exhibited the highest binding profile to the GDU-S protein at pH 4.4, but the EC50 values were estimated since the plateau was not achieved.

### • ELISA based domain mapping

For the ELISA-based domain mapping, different spike forms as antigens including S1 and its domains (domain O, A and B) and the S ectodomain (SED) were used (PEDV GDU-S strain). The antigens were coated and an anti-his tag antibody was used to detect binding by the VHH.

All the VHHs except VHH candidate 3 showed good reactivity to S1 domains and S ectodomain, indicating that they all target the S1 subunit.

### 3. Neutralisation Assay

The neutralization test uses live virus and cell culture methods to determine whether the VHH candidates can prevent viral infection *in vitro.*

Briefly, serial dilutions of the VHH candidates are mixed with a reference strain of viable virus (rPEDV-DR13-Rluc) and incubated to allow VHHs to bind and neutralize the virus. The mixtures are then inoculated onto virus-sensitive Vero cells. The neutralizing activity is observed by measuring light emission (use of Luciferase-fluorescent Reporter). Vero cells were prepared by rinsing and incubating with DMEM for 1 hour at room temperature. A 1:2.5 and a 1:12.5 dilution of cells were made and 100 µL added to each well of a 96-well plate and incubated for 24 hours at 37°C. The luciferase activity was measured at 24 hours post infection. Measurement of relative luciferase activity reflects the percentage of inhibition. VHHs of interest were isolated on the basis of their neutralising activity. The measurements were done in quadruplicate.

The neutralisation results (means of the four replicates) expressed as percentage of infection and obtained for each lead candidate are presented in tables 6 and 7 below:

**Table 6**

| | | VHH Candidate | | | | | |
|---|---|---|---|---|---|---|---|
| VHH Conc.(ug/ml) | Positive control | 1 | 2 | 3 | 4 | 5 | 6 |
| 10.00 | 14.17 | 96.90 | 169.75 | 112.55 | 175.25 | **9.72** | 279.25 |
| 3.33 | 18.24 | 97.91 | 149.68 | 126.75 | 179.5 | **10.59** | 227.25 |
| 1.11 | 16.26 | 103.12 | 137.60 | 147.50 | 152 | **14.03** | 259.50 |
| 0.37 | 24.58 | 95.39 | 167.25 | 132.23 | 177 | **21.58** | 237.43 |
| 0.12 | 25.93 | 97.30 | 108.93 | 135.18 | 146.5 | **52.28** | 220.75 |
| 0.04 | 34.70 | 87.11 | 124.63 | 142.75 | 148 | **78.63** | 181.50 |
| 0.01 | 51.50 | 63.87 | 113.28 | 134.48 | 130 | **100.20** | 122.25 |

**Table 7**

| | | VHH Candidate | | | |
|---|---|---|---|---|---|
| VHH Conc.(ug/ml) | Positive control | **7** | 8 | **9** | 10 |
| 10.00 | 14.17 | **21.30** | 71.6 | **6.20** | 93.68 |
| 3.33 | 18.24 | **35.60** | 77.05 | **8.98** | 91.73 |
| 1.11 | 16.26 | **53.70** | 66.175 | **9.61** | 89.93 |
| 0.37 | 24.58 | **97.93** | 90.025 | **19.60** | 112.65 |
| 0.12 | 25.93 | **118.95** | 106.575 | **37.85** | 85.18 |
| 0.04 | 34.70 | **128.63** | 109.6 | **64.63** | 106.03 |
| 0.01 | 51.50 | **113.03** | 96.125 | **89.83** | 96.40 |

Based on the results, some of the VHHs do not exhibit sufficient neutralizing effect compared with the positive control, whereas VHH candidates 5, 7 and 9 have demonstrated a strong neutralizing effect.

### Example 3: Selection of the lead candidates

### Cross reactivity to different PEDV strains

The inventors have designed a cross-reactivity assay to identify VHHs able to bind both non-S-INDEL and S-INDEL strains of PEDV. The following six PEDV strains were used for the cross-reactivity assay:
non-S-INDEL strains:
   - GDU / GenBank accession no. KU985230
   - FJ-9 / GenBank accession no. AGG34696
   - USA / GenBank accession no. All20255
S-INDEL strains:
   - UU / GenBank accession no. KU985229
   - DR13 / GenBank accession no. JQ023162.1
   - CV777 / GenBank accession no. AF353511

Microtiter plates were coated with the S1 polypeptide from the above six strains of PEDV (100.0 nanogram per well, diluted in PBS) and incubated overnight at 4 °C. After washes with washing buffer (PBS containing 0.05% Tween 20), the plates were blocked with blocking buffer (PBS containing 3% BSA, 0.1% Tween 20 in PBS) and then incubated with different VHH candiates diluted in PBS containing 1% BSA for 1 hour at room temperature.

After a washing step, a 1:2000 diluted HRP-conjugated Rabbit anti-Mouse IgG or HRP-conjugated Goat anti Human IgG was added and incubated at room temperature for 1 hour. After a washing step, 100 µl of tetramethylbenzidine-hydrogen peroxide substrate (TMB) was added. Plates were incubated at room temperature for 5 min and the reaction was stopped by adding 100 µl stop solution (sulfuric acid) and the reactions were measured as optical density (OD) at 450 nm using an ELISA plate reader.

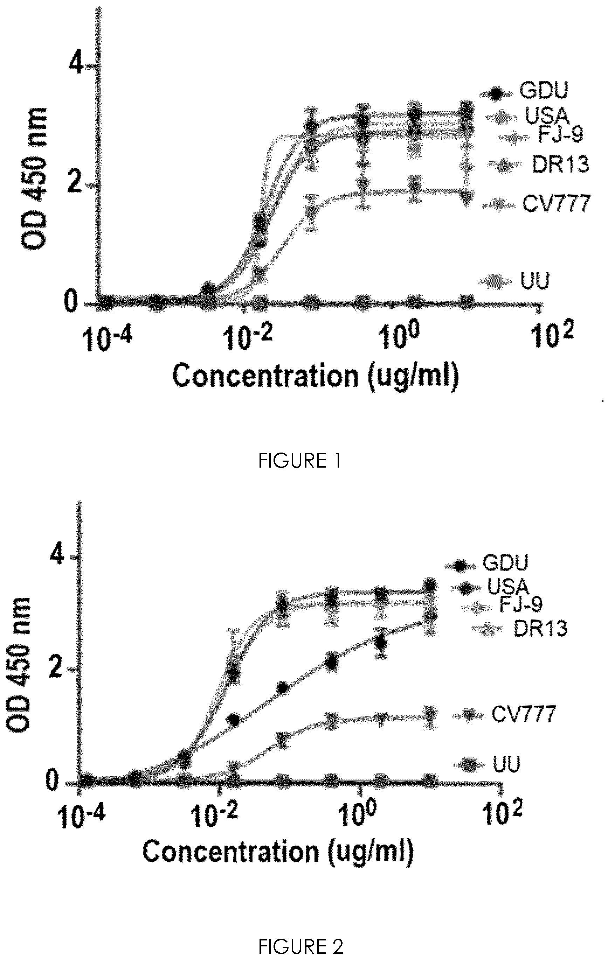

Two VHH have demonstrated the ability to cross react with S-INDEL and non-S-INDEL strains of PEDV, and the results are presented the table 8 below (mean of two replicates for each dilution) and in Figures 1 and 2 for VHH candidate 5 and 7 respectively:

**Table 8**

| | VHH candidate 5 | | |
|---|---|---|---|
| | 1:50 | 1:200 | 1:800 |
| GDU | 2.57 | 1.68 | 0.565 |
| UU | 3.12 | 2.48 | 0.645 |
| FJ-9 | 2.30 | 1.53 | 0.57 |
| USA | 3.16 | 2.34 | 0.86 |
| DR13 | 3.50 | 2.19 | 0.675 |
| CV777 | 2.39 | 1.56 | 0.54 |

| | VHH candidate 7 | | |
|---|---|---|---|
| | 1:50 | 1:200 | 1:800 |
| GDU | 2.73 | 1.75 | 0.61 |
| UU | 2.92 | 1.71 | 0.57 |
| FJ-9 | 2.73 | 2.13 | 0.40 |
| USA | 3.36 | 2.61 | 1.02 |
| DR13 | 3.33 | 2.16 | 0.71 |
| CV777 | 2.27 | 1.42 | 0.51 |

Therefore, as also depicted on Figures 1 and 2, the cross reactivity of the VHH candidates 5 and 7 is well demonstrated for both non-S-INDEL and S-INDEL strains of PEDV, this characteristic being an important advantage of the VHH according to the invention.

### Example 4: Design and characterization of bivalent VHH

Bivalent formats were designed starting from the two VHH best candidates 5 and 7 using five GS repeats as linker and characteristics are presented in table 9 below:

**Table 9**

| Bivalent reference | MW (kDa) | Concentration (mg/ml) | Protein amount (mg) | Monomer by aSEC (%) | LPS (EU/mg) |
|---|---|---|---|---|---|
| VHH5-5GS-VHH5 | 29.43 | 0.60 | 5.52 | 100 | <0.20 |
| VHH7-5GS-VHH7 | 27.18 | 0.57 | 7.07 | 100 | <0.20 |

### EC50 by ELISA at pH 7.4 and pH 4.4

The two bivalents showed specific binding to GDU-S Spike protein at pH 7.4 and 4.4. Full titration was obtained for all samples tested and the EC50 values were as presented in table 10 below:

**Table 10**

| | EC50 (nM) | |
|---|---|---|
| | pH 4.4 | pH 7.4 |
| VHH5-5GS-VHH5 | 0.471 | 0.389 |
| VHH7-5GS-VHH7 | 0.336 | 0.285 |

The two clones that combined the same VHH on both sides (VHH5-5GS-VHH5 and VHH7-5GS-VHH7) presented the lowest OD values at pH 4.4. Overall, lower KD values were obtained for the bivalent VHHs when compared to the respective single VHH.

### Example 5: Sensitivity to pancreatin digestion and PEDV binding

To test the ability of the VHHs according to the invention to maintain their binding to PEDV in conditions found in the porcine tractus, a porcin pancreatin digestion experiment was performed with the VHH candidate 7 and its bivalent VHH7-5GS-VHH7. Briefly, the experiment was set-up as follows:
- the VHH clones were diluted to 30 µM, in assay buffer (50 nM Tris-HCL, 10 mM CaCl₂ - pH 6.8).
- Porcine Pancreatin was made up at 12.5 mg/mL in 1 mM HCl (pH3.0), incubated for 90 minutes, and diluted down to 1 mg/mL in assay buffer (50mM Tris-HCl, 10mM CaCl2 - pH6.8). The pancreatin contains trypsin, chymotrypsin, amylase, elastase and lipase, ribonuclease, and protease.
- VHH : Pancreatin mix was incubated, at 37°C, for 0, 60, 120 and 180 min. For the control, no enzyme was added.
- After the incubation period, a cocktail of protease inactivators were added to tubes, for pancreatin neutralization.

The digested VHHs were either ran on SDS-PAGE or tested on an ELISA binding assay, and the ELISA set-up is as follows:
- GDU-S pike protein was coated in a Maxisorp plate at, 2 µg/ml, 4°C, O/N,
- Coated plate was blocked with 4% skimmed milk in 1x PBS,
- Undigested and pancreatin digested VHH were titrated by performing 8-steps, three-fold serial dilutions, starting at a 300 nM for VHH monovalent and 60 nM for bivalent format in 1% skimmed milk in 1x PBS, pH 7.4,
- Pancreatin digested and undigested samples were detected with MonoRab rabbit anti camelid VHH cocktail HRP ( Cat No. A02016-200) at 1:4000 dilution 1% skimmed milk in 1x PBS, pH 7.4.
- Development was performed using TMB (eBioscience, Cat. nr. 00-4201-56) and reaction stopped using 0.5 M H₂SO₄,
- O.D. was measure at 450 nm using a microplate spectrophotometer,
- ELISA buffer: 1xPBS buffer pH7.4,
- Washing buffer: 1xPBS with 0.5% Tween 20 pH 7.4.

### Results

| | | **AA sequence** | **SEQ ID NO:** |
|---|---|---|---|
| **VHH candidate 7** | **HCDR1** | FVAMA | 1 |
| | **HCDR2** | RISSTGTVTNYADSVKG | 2 |
| | **HCDR3** | VPGNLLP | 3 |
| | **Full sequence** | EVQLVESGGGLVQAGGSLRLSCAASGSLLSFVAMA WYRQVPGKEREVVARISSTGTVTNYADSVKGRFTISRD NAKKTLDLQMNSLKPEDTAVYYCNRVPGNLLPWGQ GTQVTVSS | 4 |
| **VHH7-5GS-VHH7** | **Full sequence** | EVQLVESGGGLVQAGGSLRLSCAASGSLLSFVAMA WYRQVPGKEREVVARISSTGTVTNYADSVKGRFTISRD NAKKTLDLQMNSLKPEDTAVYYCNRVPGNLLPWGQ GTQVTVSSGSGSGSGSGSEVQLVESGGGLVQAGG SLRLSCAASGSLLSFVAMAWYRQVPGKEREVVARISST GTVTNYADSVKGRFTISRDNAKKTLDLQMNSLKPEDTA VYYCNRVPGNLLPWGQGTQVTVSS | 5 |

### Sequence of the selected anti-GDU-S VHH candidate 7 and its bivalent format

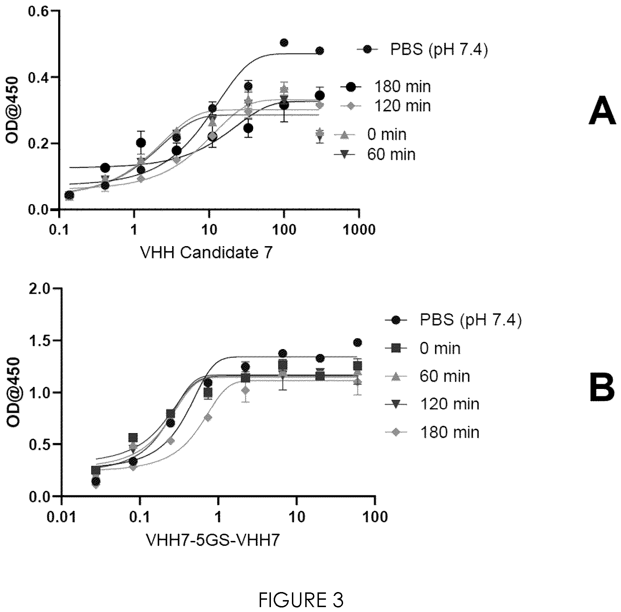

On SDS-PAGE, both monovalent VHH candidate 7 and bivalent VHH7-5GS-VHH7 showed incubation time dependent resistant to pancreatin digestion (data not shown), and advantageously, the bivalent VHH7-5GS-VHH7 exhibits potent retention of GDU-S retention of binding property to target GDU-S protein post pancreatin digestion (see Figure 3). Further, a positive correlation between SDS-PAGE and ELISA data were observed for pancreatin digested samples of VHH7-5GS-VHH7 candidate showing very slight loss of binding activity by 180min post digestion.

## Claims

1. An anti-coronavirus antibody comprising or consisting of a first heavy chain variable domain comprising:
- a CDR1 sequence comprising or consisting of FVAMA (SEQ ID NO: 1);
- a CDR2 sequence comprising or consisting of RISSTGTVTNYADSVKG (SEQ ID NO: 2) or a sequence having at least 90% identity with SEQ ID NO: 2;
- a CDR3 sequence comprising or consisting of VPGNLLP (SEQ ID NO: 3) or a sequence having at least 90% identity with SEQ ID NO: 3.

2. The anti-coronavirus antibody according to claim 1, wherein the first heavy chain variable domain comprises or consists of 
EVQLVESGGGLVQAGGSLRLSCAASGSLLSFVAMAWYRQVPGKEREVVARISSTGTVTNYADS
VKGRFTISRDNAKKTLDLQMNSLKPEDTAVYYCNRVPGNLLPWGQGTQVTVSS (SEQ ID NO: 4)
 or of a sequence having at least 90% identity with SEQ ID NO: 4.

3. The anti-coronavirus antibody according to claim 1 or 2, wherein the first heavy chain variable domain is a VHH or a nanobody.

4. The anti-coronavirus antibody according to anyone of claims 1 to 3, comprising two or more first heavy chain variable domains.

5. The anti-coronavirus antibody according to anyone of claims 1 to 4, further comprising at least one second heavy chain variable domain, preferably the second heavy chain variable domain is a VHH or a nanobody.

6. The anti-coronavirus antibody according to claim 5, wherein the second heavy chain variable domain is directed against an epitope of the coronavirus spike protein which is different from the epitope of the first heavy chain variable domain.

7. The anti-coronavirus antibody according to claim 6, wherein the first heavy chain variable domain comprises or consists of 
EVQLVESGGGLVQAGGSLRLSCAASGSLLSFVAMAWYRQVPGKEREVVARISSTGTVTNYADS
VKGRFTISRDNAKKTLDLQMNSLKPEDTAVYYCNRVPGNLLPWGQGTQVTVSSGSGSGSGSGSE
 
VQLVESGGGLVQAGGSLRLSCAASGSLLSFVAMAWYRQVPGKEREVVARISSTGTVTNYADSV
KGRFTISRDNAKKTLDLQMNSLKPEDTAVYYCNRVPGNLLPWGQGTQVTVSS (SEQ ID NO: 5) or
 of a sequence having at least 90% identity with SEQ ID NO: 5.

8. The anti-coronavirus antibody according to anyone of claims 1 to 7, which is a neutralizing antibody.

9. The anti-coronavirus antibody according to anyone of claims 1 to 8, which is an anti-PEDV antibody, in particular an anti-non-S-indel PEDV antibody.

10. A nucleic acid encoding the anti-coronavirus antibody according to anyone of claims 1 to 9.

11. A vector comprising the nucleic acid of claim 10.

12. The anti-coronavirus antibody according to anyone of claims 1 to 8, the nucleic acid according to claim 9, or the vector according to claim 10, for use as a medicament in an individual.

13. The anti-coronavirus antibody according to anyone of claims 1 to 8, the nucleic acid according to claim 10, or the vector according to claim 11, for use in the prevention or treatment of a coronavirus infection in an individual.

14. The anti-coronavirus antibody according to anyone of claims 1 to 8, the nucleic acid according to claim 10, or the vector according to claim 11, for use in the prevention or treatment of a PEDV infection.

15. The anti-coronavirus antibody, the nucleic acid, or the vector for use according to anyone of claims 12 to 14, wherein the individual is a porcine.

16. A pharmaceutical composition comprising the anti-coronavirus antibody according to anyone of claims 1 to 9, the nucleic acid according to claim 10, or the vector according to claim 11, as an active ingredient, optionally in association with at least one pharmaceutically acceptable carrier or excipient.

17. The use of the anti-coronavirus antibody according to anyone of claims 1 to 9, for the *in vitro* detection or quantification of a coronavirus, in particular a PEDV, in a biological sample.
